# EUROPEAN PATENT SPECIFICATION

(11) **EP 1 396 252 B1**
(45) Date of publication and mention of the grant of the patent: **03.01.2007**
(21) Application number: 03015560.0
(22) Date of filing: 10.07.2003
(51) Int. Cl.: A61K 6/00

(54) **Denture adhesive comprising carboxymethylcellulose and calcium sulfate**
Zahnprothesenhaftmittel enthaltend Carboxymethylcellulose und Calciumsulfat
Composition adhesive pour prothèse dentaire comprenant de la carobxymethylcellulose et du sulfate de calcium

(30) Priority: 06.09.2002 JP 2002261461
(43) Date of publication of application: 10.03.2004
(73) Proprietor: Japan Dental Supply Corporation, Chiyoda-ku, Tokyo (JP)
(72) Inventor: Muramatsu, Hiroaki, Konosu-shi, Saitama-ken (JP); Kozutsumi, Yoshio, Takasaki-shi, Gunma-ken (JP)
(74) Representative: Müller-Boré & Partner Patentanwälte

(56) References cited:
- EP-A- 1 166 744
- GB-A- 754 375
- US-A- 4 339 279
- US-A- 4 664 630

## Description

The present invention relates to an adhesion type denture adhesive that can keep good adhesiveness for a long period of time.

A denture adhesive is a material that is used for stabilizing a nonconforming denture in an oral cavity and exerts such effects that assist occlusion and mastication and facilitate conversation. The denture adhesive is classified in terms of composition into a so-called "close contact type" denture adhesive containing a vinyl acetate resin as a base component and "an adhesion type" denture adhesive containing a water soluble polymer having high adhesiveness such as karaya gum and sodium carboxymethyl cellulose as a main component.

The close contact type denture adhesive is such a denture adhesive that removes air between the denture base and an oral mucosa to exert vacuum sorption of the denture on the surface of the oral mucosa, so as to stabilize the denture with the adhesiveness thus obtained, and it generally contains a vinyl acetate resin as a main component and is in a form of a rubber-like paste having elasticity. The close contact type denture adhesive has such characteristics that the denture can be washed with water after detaching it from the oral cavity owing to the water insolubility thereof, and it can be used in the case where the gap between the denture base and the oral mucosa is relatively large, and the close contact type dental adhesive can be easily released from the oral mucosa. However, it has such disadvantages that an alcohol, which is a component of the close contact type denture adhesive, irritates the oral mucosa, the close contact type denture adhesive is liable to remain on the surface of the denture made with a resin, and the close contact type denture adhesive is difficult to be removed from the denture base after use.

On the other hand, the adhesion type denture adhesive containing a water soluble polymer as a base component, such as karaya gum and sodium carboxymethyl cellulose is such an adhesion type denture adhesive that exerts adhesiveness by swelling or dissolving the water soluble polymer through contact with saliva in the gap between the denture base and the oral mucosa, so as to attach and stabilize the denture on the oral mucosa. The adhesion type denture adhesive can be further classified in terms of the form thereof into a powder form, a paste form, which is obtained by mixing powder of the water soluble polymer with a mineral oil, such as vaseline and liquid paraffin, or a dehydrated polyhydric alcohol, such as glycerin and polyethylene glycol, and a sheet form, which is obtained by dissolving powder of the water soluble resin into a solvent, followed by subjecting to drying, such as freeze drying.

The adhesion type denture adhesive in any form is excellent in stabilizing power for the denture in comparison to the close contact type denture adhesive and has such characteristics that it brings about less uncomfortable feeling since it can be uniformly applied between the denture base and the oral mucosa to a small thickness. However, thewatersolublepolymerswollen or dissolved with an aqueous component is in the form of a sticky paste, and it has such disadvantages that the operation for removing the adhesion type denture adhesive adhered on the denture base and the oral mucosa after use is difficult, and thus the adhesion type denture adhesive adhered on the denture base and the oral mucosa is difficult to be removed. Furthermore, because the substance exerting adhesiveness is a water soluble sticky paste, which can easily run off into the oral cavity, it has such a disadvantage that the adhesiveness cannot be kept for a long period of time.

In order to solve the problem, for example, JP-A-2002-626 proposes an adhesion type denture adhesive formed by mixing an alginate salt and calcium sulfate with an adhesion type denture adhesive containing a water soluble polymer as a main component, which is excellent in cleaning property through gel effect of a gel substance formed with an alginate salt and calcium sulfate. However, the adhesion type denture adhesive using an alginate salt and calcium sulfate is excellent in adhesiveness but exerts less effect of keeping the adhesiveness for a long period of time, and it also has such a disadvantage that an alginate salt involves undeniable damaging behavior due to contamination with endotoxin originated from the raw material thereof.

An object of the invention is to provide such an adhesion type denture adhesive that is excellent in cleaning property without the use of an alginate salt and keeps adhesiveness for a long period of time while retaining the adhesiveness equivalent to the conventional adhesion type denture adhesives.

As a result of earnest investigations made by the inventors for attaining the object, it has been found that, in the case where calcium sulfate and sodium carboxymethyl cellulose are mixed with an adhesion type denture adhesive in specific amounts, gel is formed by the reaction between calcium sulfate and sodium carboxymethyl cellulose upon contact with an aqueous component, and the gel exhibits less elution of a paste of a water soluble polymer into the oral cavity while retaining cleaning property and adhesiveness equivalent to those of gel formed from an alginate salt and calcium sulfate, whereby the adhesiveness can be kept for a long period of time. Thus, the invention has been completed.

The present invention relates to an adhesion type denture adhesive using a water soluble polymer, containing 0.1 to 20% by weight of calcium sulfate and 3 to 60% by weight of sodium carboxymethyl cellulose as a sole substance for forming gel through reaction with the calcium sulfate upon contact with an aqueous component. In the denture adhesive of the present invention the water soluble polymer other than the sodium carboxymethyl cellulose is a polyacrylate salt and/or a partially alkali neutralized polyacrylate. The adhesive does not comprise alginate salt.

As the sodium carboxymethyl cellulose used in the denture adhesive of the present invention, sodium carboxymethyl cellulose that has conventionally been used in an adhesion type denture adhesive can be used without particular limitation, and it is preferably sodium carboxymethyl cellulose having a viscosity of 30 to 2, 000 cP, and more preferably 100 to 1,000 cP, in a 1% by weight aqueous solution at 25°C. The mixing amount thereof is 3 to 60% by weight, and preferably 8 to 30% by weight, based on the total amount. In the case where the mixing amount is less than 3% by weight, no effect is obtained from the gel thus obtained, and in the case where it exceeds 60% by weight, the denture adhesive is deteriorated in flowability and cannot be applied in the gap between the denture base and the oral mucosa to a small thickness.

The calcium sulfate used in the denture adhesive of the present invention may be either a dihydrated salt or a hemihydrated salt. The mixing amount thereof is 0.1 to 20% by weight, and preferably 0.3 to 8% by weight, based on the total amount of the denture adhesive. In the case where the mixing amount is less than 0.1% by weight, no effect of forming gel with the sodium carboxymethyl cellulose is obtained, and in the case where it exceeds 20% by weight, the denture adhesive is deteriorated in adhesiveness.

As the water soluble polymer used in the denture adhesive of the present invention other than the sodium carboxymethyl cellulose is a polyacrylate salt and/or a partially alkali neutralised polyacrylate. Such a water soluble polymer has conventionally been used as a base component of the denture adhesive utilizing adhesiveness obtained by swelling or dissolving the water soluble polymer, as far as gel is not formed by reaction with calcium sulfate upon contact with an aqueous component, and the effect of the present invention is obtained by mixing suitable amounts of calcium sulfate and sodium carboxymethyl cellulose with an adhesion type denture adhesive. Examples of further water soluble polymer used in the adhesion type denture adhesive other than the sodium carboxymethyl cellulose include karaya gum, gum arabic, and a methoxyethylene-maleic anhydride copolymer, which can also be used in the present invention as a mixture of two or more of them.

As the water soluble polymer other than the sodium carboxymethyl cellulose, a polyacrylate salt is used from the standpoint of adhesive force, and because a polyacrylate salt has a high pH and causes high irritation to the oral mucosa, it is preferred that the polyacrylate salt is preferably used after lowering the pH thereof by mixing with a partially alkali neutralized polyacrylate, which is a polyacrylic acid partially having alkali neutralized units.

Examples of the usable polyacrylate salt include a sodium salt, an ammonium salt, a calcium salt, a magnesium salt and an aluminum salt of polyacrylic acid, and the molecular weight thereof is generally about 20, 000 or more, and preferably 50,000 to 15,000,000. Among these salts, a sodium salt is most preferred.

In the partially alkali neutralized polyacrylate, examples of the alkali metal include sodium, potassium and lithium, and sodium is preferred. The polyacrylate and the partially alkali neutralized polyacrylate are used as the water soluble polymer other than the sodium carboxymethyl cellulose and the mixing ratio by weight of the polyacrylate salt and the partially alkali neutralized polyacrylate is preferably 1/9 to 9/1 by weight, and preferably from 4/6 to 8/2 by weight. The partially alkali neutralized polyacrylate preferably has a pH of from 5.0 to 9.0, and preferably from 5.5 to 7.5. In the case where the mixing by weight ratio or the pH is out of the ranges, there is such a tendency that a suitable pH value is difficult to be obtained with the denture.

The denture adhesive of the present invention may be in any form, such as a powder form, a paste form or a sheet form, as in the conventional adhesion type denture adhesive. In the case where it is in a paste form, it can be obtained by mixing a formulation in a powder form with a mineral oil, such as vaseline and liquid paraffin, or a dehydrated polyhydric alcohol, such as glycerin and polyethylene glycol, in an amount of 60% by weight or less, and preferably from 15 to 60% by weight, based on the total amount.

The denture adhesive of the present invention may further contain other components that do not form gel by reaction with calcium sulfate upon contact with an aqueous component after mixing with the foregoing components. Examples of the components that may be further contained include an antiseptic agent, a pH adjusting agent, an enzyme, a coloring agent and a perfume material.

The present invention will be described in detail with reference to the following examples, but it is not construed as being limited thereto.

### Example 1

The following components were mixed to obtain a denture adhesive in a paste form.

| | |
|---|---|
| Sodium polyacrylate | 30% by weight |
| Sodium carboxymethyl cellulose | 15% by weight |
| Calcium sulfate (dihydrate) | 3% by weight |
| Liquid paraffin | 52% by weight |

### Example 2

The following components were mixed to obtain a denture adhesive in a paste form.

| | |
|---|---|
| Sodium polyacrylate | 20% by weight |
| Partially sodium neutralized polyacrylate | 15% by weight |
| Sodium carboxymethyl cellulose | 10% by weight |
| Calcium sulfate (dihydrate) | 3% by weight |
| Liquid paraffin | 52% by weight |

### Example 3

The following components were mixed to obtain a denture adhesive in a paste form.

| | |
|---|---|
| Sodium polyacrylate | 10% by weight |
| Partially sodium neutralized polyacrylate | 10% by weight |
| Sodium carboxymethyl cellulose | 35% by weight |
| Calcium sulfate (dihydrate) | 3% by weight |
| Liquid paraffin | 42% by weight |

### Example 4

The following components were mixed to obtain a denture adhesive in a paste form.

| | |
|---|---|
| Sodium polyacrylate | 35% by weight |
| Sodium carboxymethyl cellulose | 10% by weight |
| Calcium sulfate (dihydrate) | 2% by weight |
| Liquid paraffin | 33% by weight |
| White vaseline | 20% by weight |

### Example 5 (not within the scope of the claim)

The following components were mixed to obtain a denture adhesive in a paste form.

| | |
|---|---|
| Karaya gum | 20% by weight |
| Sodium carboxymethyl cellulose | 25% by weight |
| Calcium sulfate (hemihydrate) | 15% by weight |
| Polyethylene glycol | 40% by weight |

### Example 6 (not within the scope of the claim)

The following components were mixed to obtain a denture adhesive in a paste form.

| | |
|---|---|
| Methoxyethylene-maleic anhydride Copolymer | 33% by weight |
| Sodium carboxymethyl cellulose | 10% by weight |
| Calcium sulfate (dihydrate) | 5% by weight |
| Liquid paraffin | 52% by weight |

### Comparative Example 1

The following components were mixed to obtain a denture adhesive in a paste form.

| | |
|---|---|
| Karaya gum | 35% by weight |
| Calcium sulfate (hemihydrate) | 15% by weight |
| Polyethylene glycol | 50% by weight |

### Comparative Example 2

The following components were mixed to obtain a denture adhesive in a paste form.

| | |
|---|---|
| Karaya gum | 35% by weight |
| Sodium carboxymethyl cellulose | 25% by weight |
| Polyethylene glycol | 40% by weight |

### Comparative Example 3

The following components were mixed to obtain a denture adhesive in a paste form.

| | |
|---|---|
| Karaya gum | 30% by weight |
| Sodium alginate (780 cP) | 15% by weight |
| Calcium sulfate (dihydrate) | 5% by weight |
| Liquid paraffin | 50% by weight |

The denture adhesive produced in the Examples and the Comparative Examples were evaluated for a duration of adhesiveness, in the following manner. The results obtained are shown in Table 1 below.

### Measurement of Duration of Adhesiveness

A denture was well washed with water, and water was wiped with tissue paper or the like.

The denture adhesive of Examples and the Comparative Examples was applied to the denture base by the ordinary method, and the denture was installed in an oral cavity with tight occlusion. Several subjects having the denture installed were subjected to ordinary life including drinking and eating, and the time when the adhesiveness of the denture adhesive was lost to fail to retain the denture base stably was recorded in 0.5 hour unit. In the case where the adhesiveness remained at the bedtime, it was considered that the duration was continued over that time.

### Measurement of Adhesion

0.2 g of the denture adhesive produced in the Examples and the Comparative Examples and 0.1 g of water were placed on an acrylic resin plate having a diameter of 10 mm and a height of 3 mm, and sandwiched with another acrylic resin plate having the same dimensions. A load of 1 kg was applied thereto for 10 seconds, and the denture adhesive extruded from the plates was removed. The acrylic resin plates then placed in a temperature of 37°C and a humidity of 100% for 10 minutes, to measure a force required for releasing the acrylic resin plates.

### Measurement of Cleaning Property

The denture adhesive produced in the Examples and the Comparative Examples was applied to a denture base by the ordinary method, and the denture was installed in an oral cavity. After 5 hours, the denture base was taken out from the oral cavity, and the easiness in removing the denture adhesive from the denture base and the oral cavity was evaluated. Cleaning and evaluation of the denture adhesive were carried out in such a manner that the denture base was lightly washed in running water with a brush for cleaning dentures, and the time required for removing the denture adhesive from the denture base was measured for evaluation.

**TABLE 1**

| | Duration of adhesiveness (hour) | Adhesion (kgf) | Cleaning property (second) |
|---|---|---|---|
| Example 1 | 12.0 | 0.60 | 20 |
| Example 2 | ≥ 14.0 | 0.65 | 15 |
| Example 3 | ≥ 13.0 | 0.62 | 20 |
| Example 4 | 11.0 | 0.61 | 20 |
| Example 5 | 10.5 | 0.58 | 25 |
| Example 6 | 10.0 | 0.55 | 25 |
| Comparative Example 1 | 5.5 | 0.58 | 60 |
| Comparative Example 2 | 5 | 0.57 | 60 |
| Comparative Example 3 | 8 | 0.65 | 20 |

It is understood from the results shown in Table 1 that the denture adhesives according to the present invention have an adhesion not lowered and excellent cleaning property without the use of an alginate salt as compared to the denture adhesive of the Comparative Example 3, which is the Example 1 disclosed in Japanese Patent Laid-Open No. 2002-626, and keep excellent cleaning property and adhesiveness for a long period of time. On the other hand, the denture adhesives of the Comparative Examples 1 and 2 forming no gel are short in duration of adhesiveness and poor in cleaning property.

As described above in detail, the denture adhesive according to the present invention, which contains specific amounts of calcium sulfate and sodium carboxymethyl cellulose as a sole substance for forming gel through reaction with the calcium sulfate upon contact with an aqueous component in an adhesive type denture adhesive using a water soluble polymer, has excellent adhesiveness from the water soluble polymer and excellent cleaning property from the gel formed through reaction of the sodium carboxymethyl cellulose and the calcium sulfate, and also maintains the adhesiveness for a long period of time. Accordingly, the present invention greatly contributes to the field of dentistry.

## Claims

1. An adhesion type denture adhesive using a water soluble polymer, containing 0.1 to 20% by weight of calcium sulfate and 3 to 60% by weight of sodium carboxymethyl cellulose as a sole substance for forming gel through reaction with the calcium sulfate upon contact with an aqueous component,
wherein the water soluble polymer other than the sodium carboxymethyl cellulose is a polyacrylate salt and/or a partially alkali neutralized polyacrylate, and
wherein the adhesive does not comprise alginate salt.

## Patentansprüche

1. Zahnprothesenhaftmittel vom Hafttyp, unter Verwendung eines wasserlöslichen Polymers, welches 0,1 bis 20 Gewichtsprozent Calciumsulfat und 3 bis 60 Gewichtsprozent Natriumcarboxymethylzellulose als eine alleinige Substanz zum Bilden eines Gels durch eine Reaktion mit dem Calciumsulfat aufgrund von Kontakt mit einer wässrigen Komponente enthält, wobei das wasserlösliche Polymer, welches von der Natriumcarboxymethylzellulose verschieden ist, ein Polyacrylatsalz und/oder ein teilweise alkalineutralisiertes Polyacrylat ist, und wobei das Haftmittel kein Alginatsalz umfasst.

## Revendications

1. Adhésif pour prothèse dentaire du type à adhérence utilisant un polymère soluble dans l'eau, contenant 0,1 à 20% en poids de sulfate de calcium et 3 à 60% en poids de carboxyméthylcellulose sodique la sous forme d'une seule substance pour former un gel par réaction avec le sulfate de calcium lors d'un contact avec un composant aqueux,
dans lequel le polymère soluble dans l'eau autre que la carboxyméthylcellulose sodique est un sel de polyacrylate et/ou un polyacrylate partiellement neutralisé par un composé alcalin et dans lequel l'adhésif ne comprend pas de sel d'alginate.
